# EUROPEAN PATENT SPECIFICATION

(11) **EP 1 592 365 B1**
(45) Date of publication and mention of the grant of the patent: **03.12.2008**
(21) Application number: 04702956.6
(22) Date of filing: 16.01.2004
(51) Int. Cl.: A61F 2/12

(54) **SOLID IMPLANT**
FESTES IMPLANTAT
IMPLANT SOLIDE

(30) Priority: 17.01.2003 US 347091
(43) Date of publication of application: 09.11.2005
(73) Proprietor: Ku, David N., Atlanta, GA 30307 (US)
(72) Inventor: Ku, David N., Atlanta, GA 30307 (US)
(74) Representative: Jones Day
(86) International application number: PCT/US2004/001110
(87) International publication number: WO 2004/066867

(56) References cited:
- US-A- 4 657 553
- US-A- 5 773 019
- US-A- 5 997 574
- US-B1- 6 231 605
- US-B1- 6 271 278
- US-B1- 6 692 527

## Description

### Field of the Invention

The present invention relates to the field of implantable prosthesis. In particular, the implants are used in a variety of plastic surgeries including but not limited to mastectomy, augmentation, or reconstruction.

### Background

Over the years, many attempts have been made to come up with an appropriate implantable prosthesis for the breast that had the look and feel of natural breasts without any harmful side effects.

Early implants were made from foams such as polyethylene and cross-linked poly vinyl alcohol ("PVA") that were hydrophobic and had little or no water content. Current commercial breast implants are silicone bags filled with either saline or silicone oil. The bag is a potential source of inflammation since the bag is made of silicone. Breast implants filled with silicone may elicit an immune response while implants filled with saline do not look or feel as natural. Further, all implants composed of an outer envelope and an inner filler material have the possibility for leaks, ruptures, or bleeding through the membrane of the envelope. Therefore, many types of fillers are being experimented with to try and create less harmful fillers as well as fillers that are less prone to leaks.

For example, U.S. Patent 6,251,137 issued to Andrews et al., introduced an implantable prosthesis comprised of synthetic triglycerides.

U.S. Patent 6,371,984 issued to Van Dyke et al., relates an implantable prosthesis filled with a keratin hydrogel.

U.S. Patent 5,407,445 issued to Tautvydas, relates to a polyoxyethylene filler.

Thus, it is evident that a variety of filler materials and bags have been proposed and patented. However, a major shortcoming of all implants composed of an envelope and an inner filler, is that the envelope will inevitably leak, bleed, or even rupture in certain instances. The present invention proposes a different solution where the implant is completely solid throughout. Being solid throughout, the implant has no fluid that could leak, bleed or lead to a rupture. A solid implant according to the preamble of claim 1 of the current invention is known from US 6 231 605. Even the problems with "gel-bleeds" have been solved. Gel-bleed is a term to describe the sticky residue that comes off an implant after it has been cut. Gel-bleeds have been associated primarily with silcone gels. However, the present invention does not "bleed" in any manner.

### Summary Of The Invention

This invention relates to a breast implant that is one solid material without a surrounding shell or bag. The device consists of a biocompatible elastomer of appropriate shape that has a modulus of elasticity that is less than 1 megaPascal, specifically, between 100 and 500 kPascal.

Accordingly, it is an object of this invention to provide a solid one-piece implant that is not prone to ruptures or leaks. The use of a biocompatible elastomer of an appropriate size and shape will enable users to get breast augmentation or reconstructive surgery without fear of rejection by the body or damage from ruptures or leaks. The elastomer further has the look and feel consistent with normal breast tissue.

### Brief Description Of The Drawings

Fig. 1 is a perspective view of a breast implant.
Fig. 2 is a cross section view of a breast implant.

### Detailed Description Of The Preferred Embodiment

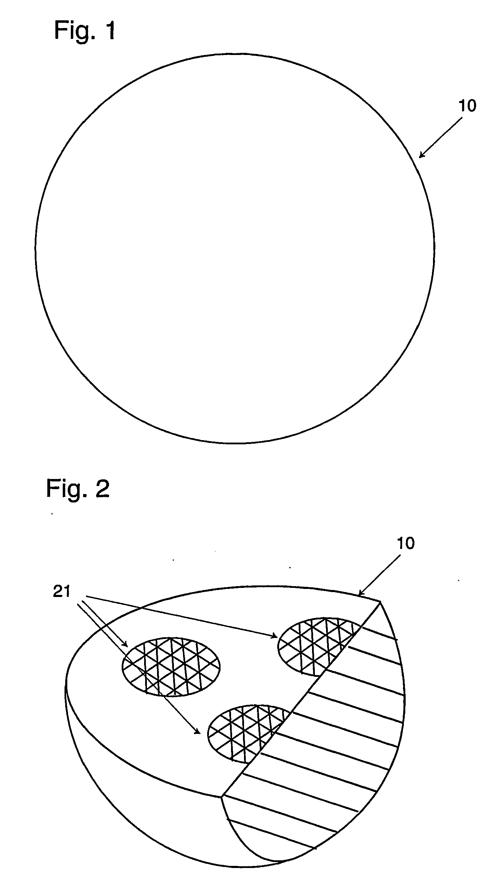

The present invention relates to a solid one-piece elastomer that is used as an implant 10. The implant 10 is suited for use in the breast but may be used in other parts of the human body. For example, addition uses contemplated are as a buttock, calf, male pectoralis or penile implant 10. Fig. 1 shows the implant 10 to be substantially circular in shape but the size and shape can vary depending upon the user's particular needs or preference. The implant 10 is made from a synthetic organic polymer that is biocompatible, compliant and has a water content greater than 5%. Biocompatibility prevents the implant 10 from being rejected by the human body. An inflammatory or immune response is typically generated when a foreign body is implanted into the human body. Molecules that are made from carbon and water are generally much more biocompatible than molecules containing silicon or other metals. The major problem with silicone has been that it is not an organic polymer, which may result in an immune response from the human body. The present invention utilizes an organic elastomer made from carbon atoms, not silicon atoms. Likewise, the body is predominantly composed of salt water. Water is clearly biocompatible. The property of hydrophyllicity (water loving) is a description of a material's affinity to water. Silicone and other implantable medical materials such as polyethylene and polytetrafluoroethylene are hydrophobic or water hating. If the implant 10 is formed from a material that contains water, then it must be hydrophilic and more likely to be biocompatible. Therefore, giving the implant 10 a water content of greater than 5% is beneficial for biocompatibility. Preferably, the solid implant 10 is made from a biocompatible elastomer with some water content such as hydrated polyurethane or polyvinyl alcohol.

In addition, the implant 10 is compliant. Compliance not only makes the implant 10 easier to work with but it gives the implant 10 a more natural look, fit and feel.

The implant 10 may be made by dissolving a polymer into saline to make a 10% weight solution. The solution is then poured into a mold in a controlled environment, preferably a globular shape, and preferably in a shape as shown in Fig. 1. The solution is then frozen to less than minus 5 degrees Celsius, preferably at a rate of less than 1 degree per minute. The implant 10 is then thawed to more than 2 degrees Celsius, preferably at a rate of less than 1 degree per minute. The freezing and thawing steps are repeated as needed to achieve solidity, preferably two times. The implant 10 is then removed from the mold in a controlled environment and placed into a package with a water barrier seal.

Fig. 2 shows a cross section of an implant 10. The form is used only for illustrative purposes, the actual size and shape of the implant 10 may vary to suit the particular needs of the user. The cross section shows that the entire implant 10 is made from one solid piece of elastomer with no separate coverings or envelopes. The elastomer is made from one component and is homogeneous throughout. A single component is easier to manufacture and provides fewer points for inflammation. However, for a given implant 10, it may be desirable to provide several components to provide a bioactive reaction such as with a drug eluting or radioactive implant 10 to treat cancer. Thus, single and multiple component implants 10 are envisioned in this invention.

The implant 10 also has a compressive modulus of elasticity between 100 and 500 kiloPascal. The solid implant 10 may have different areas with varying moduli of elasticity. The range in the modulus of elasticity allows the implant 10 to have variances that are consistent with normal breast tissue variations. For example, one part of the implant 10 may have a modulus of elasticity of 100 kiloPascals while another portion of the implant 10 may have a modulus of elasticity of 500 kiloPascals.

The implant 10 further has a tensile elongation length between 400% and 800%, which gives the implant 10 similar "stretchiness" to normal breast tissue.

The implant 10 further has a smooth, textured, or modified surface, which aids in proper placement and fixation in the body. For example, a rough texture may cause increased adherence between the implant 10 and the surrounding tissues.

A tissue fixation component 21 is combined with the solid implant 10 to enhance tissue fixation. A potential problem for a breast implant 10 made from a biocompatible material is that the implant 10 will migrate to a different anatomic location. Hence, it is useful to selectively encourage attachment at specific sites. The tissue fixation component 21 may be comprised of tabs or holes to allow the surgeon to suture the implant 10 to native body structures. Alternatively, the surface roughness and porosity may be tailored to allow for fibrotic in-growth and mechanical interlock. In another embodiment of the present invention, the material may include a biologically active agent that enhances attachment. In yet another embodiment of the present invention, a second material such as polyethylene may be molded in selective areas on the implant 10 to create fibrotic in-growth and mechanical interlock. For example, the tissue fixation component 21 may be in the form of a piece of Dacron® mesh that can be placed on the interior surface of the implant 10 to promote adhesion to the underlying chest wall or muscle fascia. Other methods may be used singly or in combination to achieve optimal attachment and these are anticipated.

The implant 10 is further designed to include a bioactive agent. A bioactive agent may be a synthetic drug or a naturally occurring molecule such as a hormone or growth factor. The implant 10 may contain such a bioactive agent to stimulate fibrotic attachment, reduce inflammation, retard cell proliferation, or many other bioactive properties depending on the agent. The invention contemplates the implant 10 that may contain the agent, not the agent itself. The bioactive agent allows for breast healing and local treatment.

The implant 10 is also given a uniform optical appearance for a variety of reasons. Since the implant 10 will be used in large part for cosmetic reasons, giving the implant 10 a pleasing look will aid in acceptance of its use. Additionally, the implant 10 is translucent which contributes to the aesthetics. Furthermore, the translucency aids in clinical examinations that look for breast lumps by shining a light from one side to the other to visualize dense lumps (trans-illumination). Breast tissue is primarily fat and has a translucency that glows under trans-illumination similar to the implant 10 described in the present invention. In contrast, other materials used for breast augmentations or reconstruction are opaque and stiff, rendering them visible under the skin.

The placing of the implant 10 in the chest wall makes a hydrophilic implant 10 preferable to hydrophobic ones such as polyethylene and cross-linked PVA that were hydrophobic and had little or no water. The hydrophilic implant 10 with a water content greater than 5% aids the implant 10 to have greater biocompatibility.

A further advantage of the present invention relates to mammograms. Mammograms enable doctors to take x-rays of the breast to check for tumors. Previous implants such as silicone implants posed problems by having a different density than the surrounding breast tissue effectively casting a shadow on the mammogram. Thus, it is very difficult to provide an accurate diagnosis using mammography with a patient that has saline or silicone breast implants. However, the present invention makes the implant 10 radiolucent due to its natural density that approximates normal breast tissue. Therefore, the present implant 10 does not hinder the use of mammograms.

The non-swelling nature of the implant 10 allows it to retain its shape and form. The implant 10 swells less than 10%.

The implant 10 contains NaCl dissolved in the water content. The salt content can range between 0% and 2.0%. Preferably, the salt content is 0.9% by weight. Normal salt is Sodium (Na) and Chloride (CI) which again aids in the biocompatible nature of the implant 10.

In yet a further embodiment of the present invention, the implant 10 may be designed to hold a cancer therapeutic agent for local treatment of cancer. The implant 10 may be designed with a chamber to hold a chemotherapeutic agent or radiological seed for brachytherapy. This chamber may be an actual void that is filled at another time or a volume that contains the agent within the solid material of the implant 10.

It is readily apparent to those skilled in the art that numerous modifications, alterations, and changes can be made without departing from the inventive concept described herein. The invention, therefore, is not to be restricted except by the appended claims.

## Claims

1. An implantable prosthesis comprising a solid space-filling implant (10) formed from a synthetic, organic and hydrophilic polymer selected from the group comprising polyvinyl alcohol and polyurethane wherein said implant (10) is biocompatible, has a modulus of elasticity between 100 kPascals and 500 kPascals, a tensile elongation length greater than 400%, has a water content greater than 5% by volume, has NaCl salt content of between 0% and 2.0% by weight, and being solid, cannot leak from manual manipulation or cuts to the surface at any point, **characterised in that** it has a tissue fixation component (21) to allow for mechanical fixation by fibrous tissue.

2. The implantable prosthesis of claim 1 where the prothesis is a breast implant and the NaCl salt content is 0.9%

3. The implantable prosthesis of claim 1 where the prothesis is a buttock prosthesis and the NaCl salt content is 0.9%.

4. The implantable prosthesis of claim 1 where the prosthesis is a calf implant and the NaCl salt content is 0.9%.

5. The breast implant of claim 2 being further defined as having a bioactive agent within the solid implant material.

6. The breast implant of claim 2 being further defined as being translucent.

7. The breast implant of claim 5 wherein the bioactive agent is a therapeutic agent for local treatment of cancer.

8. The implantable prothesis of claim 1 wherein the tissue fixation component (21) is comprised of tabs or holes to allow for surgical fixation of the prothesis to native body features.

9. The implantable prothesis of claim 1 wherein the tissue fixation component (21) is a biologically active agent that enhances attachment.

10. The implantable prosthesis of claim 1 wherein the tissue fixation component (21) is a second material molded to selective areas of the prosthesis to create fibrotic in-growth and mechanical interlock.

11. The implantable prosthesis of claim 1 wherein the tissue fixation component (21) is surface roughness and porosity of the prosthesis is tailored to allow for fibrotic in-growth and mechanical interlock.

12. The implantable prothesis of claim 10 wherein the second material is Dacron® mesh.

## Patentansprüche

1. Implantierbare Prothese, die ein festes raumfüllendes Implantat (10), das aus einem synthetischen, organischen und hydrophilen Polymer ausgewählt aus Polyvinylalkohol und Polyurethan gebildet ist, umfasst, wobei das Implantat (10) biokompatibel ist, ein Elastizitätsmodul zwischen 100 kPascal und 500 kPascal, eine Dehnlänge bei Zug größer als 400 %, einen Wassergehalt größer als 5 Vol.-%, einen NaCl-Salz-Gehalt zwischen 0 und 2,0 Gew.-% aufweist und fest ist, bei manueller Handhabung oder Schnitten an beliebigen Punkten der Oberfläche nicht leckt, **gekennzeichnet dadurch, dass** es eine Gewebefixierungskomponente (21) aufweist, die mechanische Fixierung durch Fasergewebe ermöglicht.

2. Implantierbare Prothese nach Anspruch 1, wobei die Prothese ein Brustimplantat ist und der NaCl-Salz-Gehalt 0,9 % beträgt.

3. Implantierbare Prothese nach Anspruch 1, wobei die Prothese eine Gesäßprothese ist und der NaCl-Salz-Gehalt 0.9 % beträgt.

4. Implantierbare Prothese nach Anspruch 1, wobei die Prothese ein Wadenimplantat ist und der NaCl-Salz-Gehalt 0,9 % beträgt.

5. Brustimplantat nach Anspruch 2, das ferner einen bioaktiven Wirkstoff im festen Implantatsmaterial aufweist.

6. Brustimplantat nach Anspruch 2, das ferner transluzent ist.

7. Brustimplantat nach Anspruch 5, wobei der bioaktive Wirkstoff ein therapeutischer Wirkstoff zur lokalen Behandlung von Krebs ist.

8. Implantierbare Prothese nach Anspruch 1, wobei die Gewebefixierungskomponente (21) Laschen oder Löcher umfasst, um chirurgisches Fixieren der Prothese an nativen Körperteilen zu ermöglichen.

9. Implantierbare Prothese nach Anspruch 1, wobei die Gewebefixierungskomponente (21) ein biologisch aktiver Wirkstoff ist, der Anbindung verstärkt.

10. Implantierbare Prothese nach Anspruch 1, wobei die Gewebefixierungskomponente (21) ein zweites Material ist, das an selektive Bereiche der Prothese angeformt ist, um ein Einwachsen in Fasern und mechanisches Ineinandergreifen zu erzeugen.

11. Implantierbare Prothese nach Anspruch 1, wobei die Gewebefixierungskomponente (21) Oberflächenrauigkeit ist und die Porösität der Prothese angepasst ist, um das Einwachsen in Fasern und mechanisches Ineinandergreifen zu ermöglichen.

12. Implantierbare Prothese nach Anspruch 10, wobei das zweite Material Dacron ® Mesh ist.

## Revendications

1. Une prothèse implantable comprenant un implant solide remplissant l'espace (10) formé par un polymère synthétique, organique et hydrophile sélectionné du groupe comprenant l'alcool de polyvinyle et le polyuréthane, ledit implant (10) étant biocompatible, ayant une constance élastique entre 100 kPa et 500 kPa, une longueur d'élongation de traction au-dessus de 400 %, un contenu en eau au-dessus de 5 % vol., un contenu en sel de NaCl entre 0 et 2,0 % en poids et étant solide, qui ne fuit pas en cas de manipulation manuelle ou coupures de la surface à un point quelconque, **caractérisé en ce que** l'élément de fixation tissulaire (21) permet la fixation mécanique par le tissu fibreux.

2. La prothèse implantable selon la revendication 1, la prothèse étant un implant mammaire et le contenu en sel de NaCl de 0,9 %.

3. La prothèse implantable selon la revendication 1, la prothèse étant une prothèse de fesses et le contenu en sel de NaCl de 0,9 %.

4. La prothèse implantable selon la revendication 1, la prothèse étant un implant de mollet et le contenu en sel de NaCl de 0,9 %.

5. L'implant mammaire selon la revendication 2 étant en outre défini comme ayant un agent bioactif au sein du matériau solide de l'implant.

6. L'implant mammaire selon la revendication 2 étant en outre défini comme étant translucide.

7. L'implant mammaire selon la revendication 5, l'agent bioactif étant destiné au traitement local de cancer.

8. La prothèse implantable selon la revendication 1, l'élément de fixation tissulaire (21) consistant de boucles ou lacunes pour permettre une fixation chirurgicale de la prothèse auprès des parties du corps natives.

9. La prothèse implantable selon la revendication 1, l'élément de fixation tissulaire (21) étant un agent biologiquement actif qui renforce la liaison.

10. La prothèse implantable selon la revendication 1, l'élément de fixation tissulaire (21) étant un deuxième matériau dont la forme est adaptée à des sections sélectionnées de la prothèse afin de créer une intégration fibreuse et un engagement mécanique.

11. La prothèse implantable selon la revendication 1, l'élément de fixation tissulaire (21) étant la rugosité de surface et la porosité de la prothèse étant de sorte qu'une intégration fibreuse et un engagement mécanique soient possibles.

12. La prothèse implantable selon la revendication 10, le deuxième matériau étant Dacron® Mesh.
